# EUROPÄISCHE PATENTSCHRIFT

(11) **EP 1 359 852 B1**
(45) Veröffentlichungstag und Bekanntmachung des Hinweises auf die Patenterteilung: **21.07.2004**
(21) Anmeldenummer: 02719750.8
(22) Anmeldetag: 01.02.2002
(51) Int. Cl.: A61B 17/16, A61F 2/46, A61B 19/00, A61B 17/17

(54) **NAVIGATIONSKONTROLLHILFESYSTEM**
AUXILIARY SYSTEM FOR NAVIGATION CONTROL
SYSTEME D'ASSISTANCE AU CONTROLE D'EXPLORATION

(30) Priorität: 12.02.2001 DE 10106655
(43) Veröffentlichungstag der Anmeldung: 12.11.2003
(73) Patentinhaber: ESKA Implants GmbH & Co., 23556 Lübeck (DE)
(72) Erfinder: GRUNDEI, Hans, 23558 Lübeck (DE); NASSUTT, Roman, 23879 Mölln (DE)
(74) Vertreter: Fuchs Mehler Weiss & Fritzsche
(86) Internationale Anmeldenummer: PCT/EP2002/001063
(87) Internationale Veröffentlichungsnummer: WO 2002/064042

(56) Entgegenhaltungen:
- EP-A- 0 327 509
- EP-A- 0 904 741
- DE-C- 10 012 042
- US-A- 6 102 915

## Beschreibung

Die vorliegende Erfindung betrifft ein System zur Vorbereitung und Durchführung einer Ausfräsung eines Acetabulums im natürlichen Hüftbeckenknochen im Rahmen einer endoprothetischen Versorgung eines Patienten im Hüftbereich unter Einsatz einer künstlichen Gelenkpfanne.

Die vorbereitende Ausfräsung des Acetabulums ist relevant im Hinblick auf den späteren Sitz der Beckenpfanne in dem ausgefrästen Acetabulum. Grundsätzlich ist man bemüht, die Ausfräsung so zu gestalten, daß eine Luxation des Hüftgelenkes möglichst vermieden wird, wobei die Luxationsneigung bei stark gebeugten Beinen am größten ist. Maßgeblichen Einfluß auf die Vermeidung einer Luxation ist der sogenannte Beckeneingangswinkel und der Antekurvationswinkel. Der Beckeneingangswinkel variiert zwischen 30° und 45°, der Antekurvationswinkel liegt im Bereich von ca. 8° bis 15°. Bei einem Beckeneingangswinkel von 45° überdeckt die künstliche Gelenkpfanne die künstliche Gelenkkugel am weitesten, so daß eine Luxation des *Gelenks* unwahrscheinlich ist. Letztendlich obliegt die Auswahl der entsprechenden Winkel dem Operateur, der aufgrund seiner Erfahrung eher zu kleineren oder zu größeren Winkeln tendiert.

Generell ist der Antekurvationswinkel und der Beckeneingangswinkel patientenunabhängig. Gleichwohl besteht Bedarf dafür, daß der Operateur von den patientenunabhängigen Normalwerten abweicht, um den Beckeneingangsoder Antekurvationswinkel oder beide zu variieren. Hierbei stellt sich als großes Problem, eine in der Operationsplanung für optimal empfundene Winkelkombination in die Praxis umzusetzen, daß heißt also, daß Acetabulum mit einem Fräswerkzeug mit den gewählten Winkeln auszufräsen. Dieses Problem stellt sich allein schon aufgrund der operationsbedingten Unübersichtlichkeit und der räumlichen Enge im Operationsbereich.

Vor diesem Hintergrund ist es die Aufgabe der vorliegenden Erfindung, ein eingangs genanntes System anzugeben, welches ein sehr genaues und winkeltreues Ausfräsen eines Acetabulums mit frei wählbarem Beckeneingangswinkel und Antekurvationswinkel ermöglicht.

Gelöst wird diese Aufgabe durch ein eingangs genanntes System mit den weiteren Merkmalen des Anspruchs 1. Weitere vorteilhafte Weiterbildungen ergeben sich aus den Unteransprüchen.

Grundsätzlich besteht das erfindungsgemäße System aus vier Komponenten, nämlich einer Patientenauflage, einer Kalibrierhilfe, einem Fräswerkzeug und einem Peilgerät.

Die Patientenauflage dient dazu, einen Patienten in einer definierten räumlichen Lage während der Operation zu lagern. Es ist nämlich unerläßlich, daß der Patient in normierter Lage bezüglich der Vertikalen und der Horizontalen liegt, damit ein winkelgenaues Einstellen des Fräswerkzeuges und damit winkeltreues Ausfräsen des Acetabulums ermöglicht wird.

Als zweite Komponente ist eine sogenannte Kalibrierhilfe vorgesehen, die mit der erwähnten Patientenauflage unmittelbar verbunden ist und die aus einer um die Vertikale und um die Horizontale verschwenkbare Haltevorrichtung besteht. Dabei ist die Haltevorrichtung in der gewünschten Position arretierbar. Mit der Kalibrierhilfe lassen sich der Beckeneingangswinkel und der Antekurvationswinkel in bezug auf die Vertikale bzw. Horizontale voreinstellen, und zwar winkeltreu, da der Patient in normierter Lage auf der Patientenauflage liegt.

Als dritte Komponente ist ein Fräswerkzeug zum Ausfräsen des Acetabulums vorgesehen. Dieses ist im Blickfeld des Operateurs mit einer elektronischen Empfangs- und Anzeigeeinheit ausgestattet, welches die momentane räumliche Lage der Längsachse des Fräswerkzeuges in bezug auf ein Ortungssignal in Form eines Strahlungsfeldes erfaßt und zu einer optischen Anzeige bringt.

Schließlich ist ein Peilgerät vorgesehen, welches das erwähnte Ortungssignal in Richtung auf die Empfangs- und Anzeigeeinheit aussendet, welches es dieser ermöglicht, die räumliche Lage des Fräswerkzeuges in bezug auf das Peilgerät zu ermitteln und zur Anzeige zu bringen.

Die erwähnte Haltevorrichtung der Kalibrierhilfe weist nun eine Aufnahme für das Fräswerkzeug auf, in welche es nach individueller Einstellung des gewünschten Antekurvations- und Beckeneingangswinkels durch Verschwenken der Haltevorrichtung setzbar ist. Hierdurch nimmt das Fräswerkzeug mit seiner Längsachse die gewünschte räumliche Orientierung mit den gewünschten Winkeln für den Einsatz in situ ein. Dieser räumliche Orientierung wird nun als Referenzposition durch Betätigung eines Set-Schalters an der Empfangsund Anzeigeeinheit durch Abspeichern der entsprechend empfangenen Werte des Ortungssignals vom Peilgerät gesichert. Nach Einstellung dieses Referenzwertes ist das Fräswerkzeug nun nach Entnahme aus der Aufnahme der Haltevorrichtung unter optischer Anzeige in die voreingestellte räumliche Orientierung am Acetabulum rückführbar.

Mit anderen Worten stellt der Operateur den gewünschten Antekurvationswinkel und den gewünschten Beckeneingangswinkel patientenindividuell an der Haltevorrichtung ein und arretiert diese dann.

Hierbei liegt das Fräswerkzeug vorzugsweise in der Aufnahme, so daß der Operateur bei diesen Einstellarbeiten schon eine Vorstellung für die räumliche Orientierung des Fräswerkzeuges während der späteren Ausfräsung des Acetabulums erhält. Danach betätigt der Operateur den erwähnten Set-Schalter an der Empfangs- und Anzeigeeinheit, welche diese räumliche Orientierung als Referenzposition abspeichert. Nun nimmt der Operateur das Fräswerkzeug aus der Aufnahme der Haltevorrichtung heraus und führt es an das freigelegte Acetabulum heran. Das ortsfeste Peilgerät sendet dabei weiterhin das Ortungssignal in Richtung auf den Operationsbereich aus, welches von der Empfangseinheit empfangen und ausgewertet wird, dahingehend, ob die momentane räumliche Orientierung von der als Referenzposition abgespeicherten räumlichen Orientierung abweicht oder mit ihr übereinstimmt. Bei Übereinstimmung der räumlichen Orientierung nimmt das Fräswerkzeug also exakt die räumliche Orientierung ein, die es innehatte, als es noch in der Aufnahme der Haltevorrichtung abgelegt war und der Set-Schalter bedient wurde. Der Operateur kann in diesem Falle unter ständiger Beobachtung der Anzeige das Fräswerkzeug an das Acetabulum heranführen und mit der exakten Ausrichtung des Acetabulums ausfräsen.

Weicht die räumliche Orientierung des Fräswerkzeuges von der gewünschten ab, signalisieren die permanent im Blickfeld des Operateurs liegenden Anzeigen die Richtung, in welche das Fräswerkzeug verschwenkt werden muß, damit die Referenzposition wieder eingenommen wird.

Mit großer Genauigkeit kann der Operateur also die patientenindividuell eingestellten Winkel beim Ausfräsen einhalten, was dem späteren Wohlbefinden des Patienten und der Stabilität der künstlichen Beckenpfanne im Beckenknochen zugute kommt.

In bevorzugter Ausführung umfaßt die Patientenauflage als erste Komponente des Systems einen Operationstisch, auf welchem der Patient zu liegen gebracht wird, wenigstens eine exakt senkrecht am Operationstisch geführte Führungsstange, an welcher die Kalibrierhilfe angeordnet und in der gewünschten Position arretierbar ist, sowie Beckenpelotten, welche dazu dienen, das Becken des Patienten während der Operation zu umgreifen und in Lage zu halten.

Von entscheidender Bedeutung ist die exakt senkrechte Ausrichtung der Führungsstange am Operationstisch, damit dann die an ihr geführte Haltevorrichtung exakt den gewünschten Antekurvationswinkel am Beckeneingangswinkel in bezug auf die Horizontale bzw. Vertikale einnehmen kann. Die Beckenpelotten sorgen dafür, daß das Becken des Patienten während der Operation in Lage bleibt.

Die Kalibrierhilfe umfaßt gemäß einer bevorzugten Ausführungsform einen ersten Haltearm, der um die Vertikalachse der wenigstens einen Führungsstange verschwenkbar ist, und einen zweiten Haltearm, der mit seinem einen Ende an den ersten Haltearm angelenkt ist und unabhängig von diesem verschwenkbar ist und dessen anderes Ende als Aufnahme für das Fräswerkzeug ausgebildet ist.

Mit dem um die Vertikalachse verschwenkbaren ersten Haltearm kann der Antekurvationswinkel eingestellt werden, wohingegen der Beckeneingangswinkel mit dem zweiten Arm eingestellt werden kann.

Besonders bevorzugt weist die Kalibrierhilfe zwei Skalen auf, welche die Abweichungen der Aufnahme der Kalibriervorrichtung von der Vertikalen und der Horizontalen, also den Antekurvationswinkel und den Beckeneingangswinkel anzeigt. Hierdurch ist der Operateur in der Lage, diese Winkel voreinzustellen, um danach das Fräswerkzeug in die Aufnahme der Kalibrierhilfe zu legen und gegebenenfalls noch feinere Korrekturen vorzunehmen und dann den Set-Schalter an der Empfangsvorrichtung zu betätigen und die so eingenommene räumliche Lage des Fräswerkzeuges als Referenzposition festzulegen. Alternativ hierzu kann der Operateur die beiden Winkel mit Hilfe der Skalen einstellen, die Haltevorrichtung arretieren und das Fräswerkzeug wiederum in die Aufnahme setzen und ohne weitere Korrekturen die Referenzposition abspeichern.

Gemäß einer besonders bevorzugten Ausführungsform zeigt die elektronische Empfangs- und Anzeigeeinheit des Fräswerkzeuges die momentanen Abweichungen seiner räumlichen Orientierung von der abgespeicherten Referenzposition sowie mittels eines optischen Signals die Richtung der vorzunehmenden Korrekturbewegungen, um in die gewünschte Referenzposition zu gelangen, an.

Auch hier ist von entscheidender Bedeutung, daß die Anzeige stets im Blickfeld des Operateurs liegt. Das Betrachten eines entsprechenden Signals, beispielsweise auf einem separaten Monitor entfällt und dadurch die Hinwendung zu einem anderen als dem Operationsort. Hierdurch wird die Sicherheit und die Genauigkeit der Operation erhöht.

Besonders bevorzugt ist die Ausführungsform, gemäß der das vom Peilgerät ausgesandte Ortungssignal ein Infrarotstrahlungsfeld ist und die Empfangs- und Anzeigeeinheit wenigstens zwei Infrarotsensoren aufweist, mit deren Hilfe die räumliche Lage in bezug auf das Strahlungsfeld ermittelt wird. Weicht die räumliche Orientierung des Fräswerkzeuges und damit der Empfangs- und Anzeigeeinheit von der Referenzposition ab, wird das Strahlungsfeld in dem einen Sensor entsprechend abschattiert, wohingegen der zweite Infrarotsensor verstärkt bestrahlt wird. Aus dieser Differenz ermittelt dann die Elektronik der Empfangs-Einheit die Abweichung von der Referenzposition und zeigt diese beispielsweise mit einer Reihe von LED's an, wobei die Richtung ebenfalls angezeigt werden kann, in welche das Fräswerkzeug bewegt werden muß, um mehr an die Referenzposition zu gelangen. Die Anzeige kann beispielsweise aus einem Kreuz aus LED's bestehen, wobei die Leuchtelektrode am Schnittpunkt beider Balken aufleuchtet, wenn das Fräswerkzeug die Referenzposition einnimmt. Abweichungen hiervon werden signalisiert durch die weiteren Leuchtelektroden, wobei die jeweils weiter außen liegenden Leuchtdioden aufleuchten, wenn die Abweichung stärker ist, und die Leuchtelektroden nahe an der zentralen Leuchtdiode dann aufleuchten, wenn die Referenzposition fast erreicht ist.

Die Erfindung wird nun anhand eines Ausführungsbeispiels näher erläutert. Hierbei zeigt:
- Fig. 1: die schematische Ansicht auf das System vom Fußende eines Operationstisches her gesehen,
- Fig. 2: die Ansicht mit einem auf dem Operationstisch liegenden Patienten, und
- Fig. 3: die Aufsicht auf die Empfangs- und Anzeigeeinheit.

Nachfolgend sind gleiche Teile mit den selben Bezugszeichen versehen.

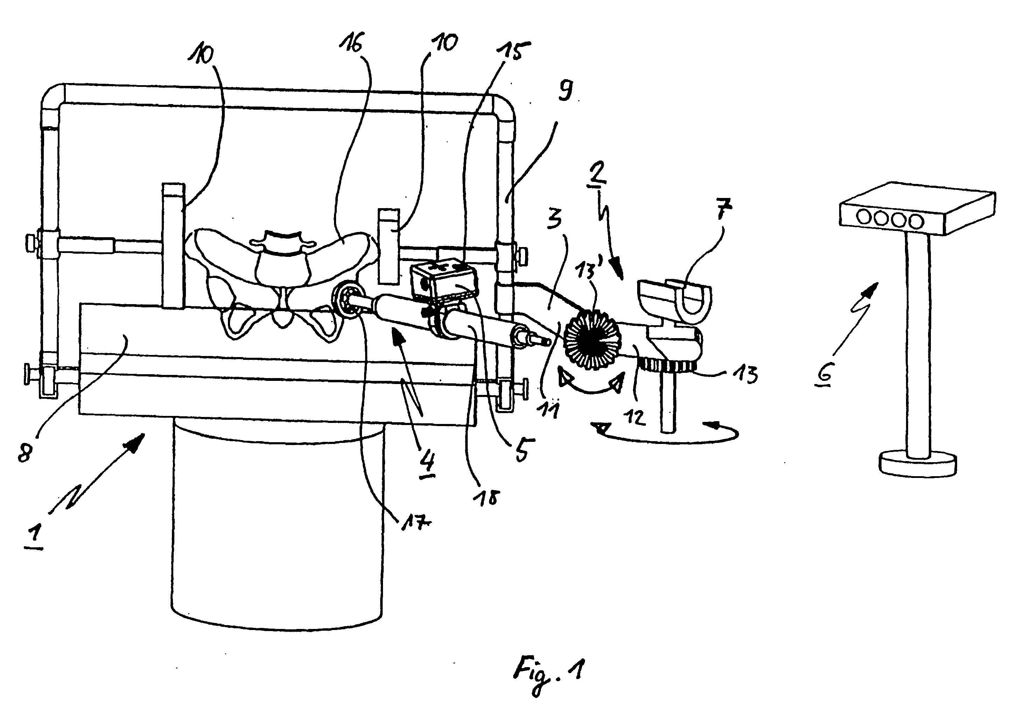

Einen ersten Überblick verschafft Fig. 1. Daran erkennbar ist die Patientenauflage 1, die vorliegend einen Operationstisch 8, eine daran exakt senkrecht geführte Führungsstange 9 sowie Beckenpelotten 10 umfaßt. Der Patient ist in Fig. 1 charakterisiert durch ein Modell 16 eines Beckens. Dieses ist eingefaßt von den Beckenpelotten 10, um das Becken 16 in eine eindeutige Lage auf dem Operationstisch 8 zu bringen und während der Operation zu halten.

Der Operationstisch 8 und die exakt senkrecht daran angebrachte Führungsstange 9 spannen ein kartesisches Koordinatensystem auf.

Mit der Führungsstange 9 ist eine Kalibrierhilfe 2 verbunden, die aus einer um die Vertikale und um die Horizontale schwenkbaren Haltevorrichtung 3 besteht. Diese ist in jeder gewünschten Position arretierbar.

Darüber hinaus ist Bestandteil des Systems ein Fräswerkzeug 4, von welchem der kopfseitige Fräskopf 17 sowie der Halter 18 abgebildet sind. Der Fräskopf 17 dient schlußendlich zur Ausfräsung des Acetabulums im Becken 16. Nicht dargestellt ist die Antriebseinheit für das Fräswerkzeug 4, welches an seinem proximalen Ende ankoppelbar ist. Im Blickfeld des Operateurs weist das Fräswerkzeug 4 eine elektronische Empfangs- und Anzeigeeinheit 5 auf. Diese Empfangs- und Anzeigeeinheit 5 ist in der Lage, die momentane räumliche Lage des Fräswerkzeugs 4 in bezug auf ein Ortungssignal zu erfassen und zu einer optischen Anzeige zu bringen.

Das Ortungssignal, welches bevorzugt ein Infrarotstrahlungsfeld ist, wird erzeugt von einem Peilgerät 6. Das Peilgerät 6 wird ortsfest im Operationssaal so aufgestellt, daß der Operationsbereich in jedem Fall zuverlässig bestrahlt wird. Die Empfangseinheit 5 verfügt über mehrere Sensoren, welche die Infrarotstrahlen vom Peilgerät 6 empfangen und aufgrund der jeweiligen Abschattierung bei einer bestimmten räumlichen Orientierung des Fräswerkzeugs rückschließen auf die Abweichungen von der Referenzposition. Die Referenzposition wird, wie nachfolgend beschrieben, bestimmt und festgelegt:

Der Operateur verschwenkt den Haltearm 11 der Kalibrierhilfe 2 um die Vertikalachse der Führungsstange 9 solange, bis der gewünschte Antekurvationswinkel erreicht ist. Hierbei kann die Skala 13 eine Hilfestellung leisten. Durch Verschwenken des zweiten Haltearms 12, der an dem ersten Haltearm 11 an dessen Ende angelenkt ist, kann der Beckeneingangswinkel eingestellt werden, vorzugsweise wieder unter Zuhilfenahme einer Skala 13'. Nun legt der Operateur das Fräswerkzeug 4 in die Aufnahme 7, die am zweiten Haltearm 12 der Kalibrierhilfe 2 ausgebildet ist. Nach dem Einlegen des Fräswerkzeuges 4 in die Aufnahme 7 hat die Längsachse des Fräswerkzeuges 4 exakt die Orientierung, welche es haben soll, wenn das Fräswerkzeug 4 an das Acetabulum herangeführt wird und der Fräskopf 17 das Acetabulum ausfräst. Nach Ablage des Fräswerkzeuges 4 in der Aufnahme 7 betätigt der Operateur einen Set-Schalter 15, wonach dann das Empfangsgerät 5 mit seiner Elektronik die dann auf seine Sensoren auftreffenden Infrarotstrahlen auswertet, daraus Signale generiert und diese als Referenzsignale abspeichert. Diese Referenzsignale korrespondieren mit der Referenzposition des Fräswerkzeuges 4 im Raum, welche das Fräswerkzeug 4 beim Ausfräsen des Acetabulums einzunehmen hat.

Nun entnimmt der Operateur das Fräswerkzeug 4 aus der Aufnahme 7 und führt es hin zum Beckenbereich des Patienten. Eine optische Anzeige führt den Operateur so, daß er in seinem Blickfeld exakt die momentanen Abweichungen der Orientierung der Längsachse des Fräswerkzeuges 4 von der Referenzposition erkennen kann. Mithin generiert das Empfangs- und Anzeigegerät 5 eine optische Anzeige, die den Operateur hin zur richtigen Position, d.h. zur Referenzposition, hinführt.

Mit dem System findet also eine Überführung einer definierten räumlichen Orientierung des Fräswerkzeuges 4 hin zum Operationsbereich statt.

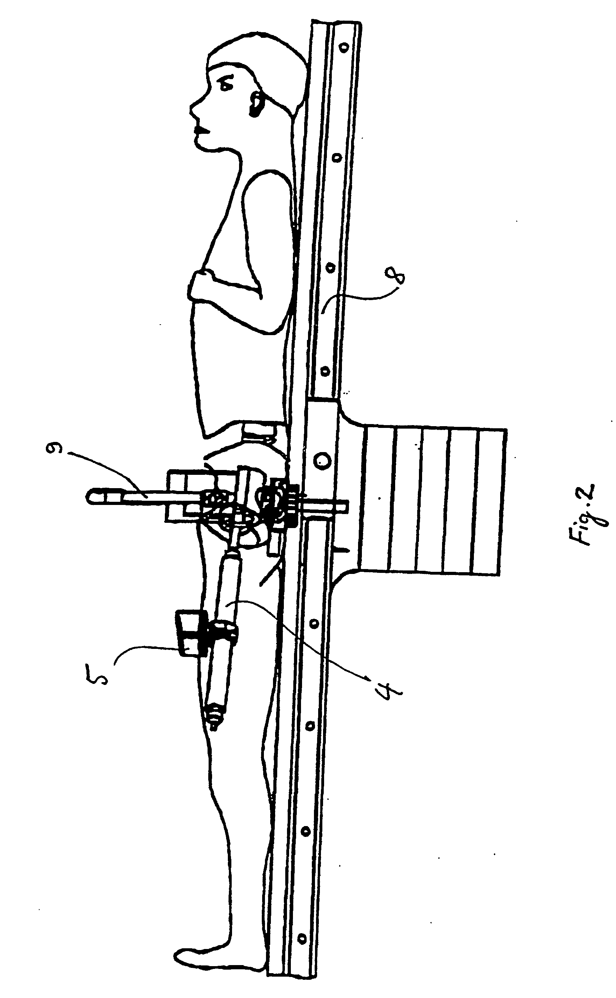

Fig. 2 zeigt einen auf den Operationstisch 8 liegenden Patienten. Fig. 2 dient zur Abrundung des Bildes. Deutlich erkennbar ist das Fräswerkzeug 4 mit daran angebrachter elektronischer Empfangs- und Anzeigeeinheit 5.

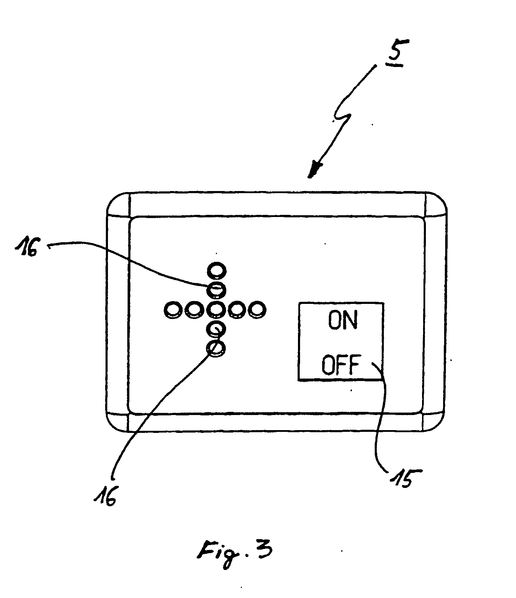

Fig. 3 zeigt Details der elektronischen Empfangs- und Anzeigeeinheit 5. Der Set-Schalter 15 ist bereits erwähnt worden. Er wird bedient, wenn das Fräswerkzeug 4 die gewünschte, patientenindividuell einstellbare räumliche Orientierung in der Kalibrierhilfe 2 eingenommen hat, woraufhin die Winkelpositionen der Sensoren in der Empfangseinheit 5 in bezug auf das Ortungssignal vom Peilgerät 6 ausgewertet werden und die entsprechenden Signale abgespeichert werden.

Entnimmt nun der Operateur das in seiner räumlichen Orientierung kalibrierte Fräswerkzeug 4 aus der Aufnahme 7, stellen die Sensoren etwaige Abweichungen von den abgespeicherten Signalen fest und steuern dementsprechend andere Leuchtdioden 16 im vorliegenden Falle an. Vorliegend ist ein kreuzförmiges Feld von Leuchtdioden 16 als Anzeige vorgesehen, wobei die Leuchtdiode im Kreuzungspunkt beider Balken jene ist, welche angesteuert wird, wenn sich das Fräswerkzeug 4 in der Referenzposition befindet. Bei Abweichungen des Antekurvationswinkels leuchten die Leuchtdioden im waagerechten Balken des Feldes auf. Bei Abweichungen im Beckeneingangswinkel leuchten die Leuchtdioden im senkrechten Balken auf. Bei Überlagerung von Abweichungen wird je eine Leuchtdiode im waagerechten und eine im senkrechten Balken angesteuert. Auf diese Weise wird es dem Operateur ermöglicht, das Fräswerkzeug 4 schnell in die patientenindividuell eingestellte Referenzposition zu bringen und die Ausfräsung des Acetabulums vorzunehmen.

## Patentansprüche

1. System zur Vorbereitung und Durchführung einer Ausfräsung eines Acetabulums im natürlichen Hüftbeckenknochen mit wählbarem, definierten Beckeneingangswinkel und Antekurvationswinkel im Rahmen einer endoprothetischen Versorgung eines Patienten im Hörbereich, aufweisend
- eine Patientenauflage (1), mittels derer ein Patient in einer definierten räumlichen Lage lagerbar ist,
- eine Kalibrierhilfe (2), die mit der Patientenauflage (1) unmittelbar verbunden ist und aus einer um die Vertikale und um die Horizontale schwenkbare Haltevorrichtung (3) besteht, wobei die Haltevorrichtung (3) in der gewünschten Position arretierbar ist,
- ein Fräswerkzeug (4) zum Ausfräsen des Acetabulums, welches im Blickfeld des Operateurs mit einer elektronischen Empfangsund Anzeigeeinheit (5) ausgestattet ist, welches die momentane räumliche Lage der Längsachse des Fräswerkzeuges (4) in bezug auf ein Ortungssignal in Form eines Strahlungsfeldes erfaßt und zu einer optischen Anzeige bringt,
- ein Peilgerät (6), welches das Ortungssignal in Richtung auf die Empfangs- und Anzeigeeinheit (5) aussendet, welches es dieser ermöglicht, die räumliche Lage des Fräswerkzeuges (4) in bezug auf das Peilgerät (6) zu ermitteln und zur Anzeige zu bringen,
wobei die Haltevorrichtung (3) der Kalibrierhilfe (2) eine Aufnahme (7) für das Fräswerkzeug (4) aufweist, in welche dieses nach individueller Einstellung des gewünschten Antekurvations- und Beckeneingangswinkels durch Verschwenken der Haltevorrichtung (3) setzbar ist, so daß das Fräswerkzeug (4) mit seiner Längsachse die gewünschte räumliche Orientierung für den Einsatz in situ einnimmt, wonach diese räumliche Orientierung als Referenzposition durch Betätigung eines Set-Schalters (15) an der Empfangs- und Anzeigeeinheit (5) durch Abspeichem der entsprechend empfangenen Werte.des Ortungssignals vom Peilgerät (6) abgespeichert wird, und wobei nach Einstellung dieses Referenzwertes das Fräswerkzeug (4) nach Entnahme aus der Aufnahme (7) unter optischer Anzeige in die voreingestellte räumliche Orientierung am Acetabulum rückführbar ist.

2. System nach Anspruch 1, bei dem die Patientenauflage (1) umfaßt:
- einen Operationstisch (8), auf welchem der Patient zu liegen gebracht wird,
- wenigstens eine exakt senkrecht am Operationstisch geführte Führungsstange (9), an der die Kalibrierhilfe (2) angeordnet und in der gewünschten Position arretierbar ist,
- Beckenpelotten (10), welche dazu dienen, das Becken des Patienten während der Operation zu umgreifen und in Lage zu halten.

3. System nach Anspruch 1 oder 2, bei dem die Kalibrierhilfe (2) umfaßt:
- einen ersten Haltearm (11), der um die Vertikalachse der wenigstens einen Führungsstange (9) verschwenkbar ist, und
- einen zweiten Haltearm (12), der mit seinem einen Ende an dem ersten Haltearm (1) angelenkt ist und unabhängig von diesem verschwenkbar ist und dessen anderes Ende als Aufnahme (7) für das Fräswerkzeug (4) ausgebildet ist.

4. System nach einem der Ansprüche 1 bis 3, bei dem die Kalibrierhilfe (2) zwei Skalen (13, 13') aufweist, welche die Abweichungen der Aufnahme der Kalibrierhilfe (2) von der Vertikalen und der Horizontalen anzeigt.

5. System nach einem der Ansprüche 1 bis 4, bei dem die elektronische Empfangs- und Anzeigeeinheit (5) des Fräswerkzeuges (4) die momentanen Abweichungen seiner räumlichen Orientierung von der abgespeicherten Referenzposition anzeigt sowie mittels eines optischen Signals die Richtung der vorzunehmenden Korrekturbewegungen, um in die gewünschte Referenzposition zu gelangen, anzeigt.

6. System nach einem der Ansprüche 1 bis 5, bei dem das vom Peilgerät (6) ausgesandte Ortungssignal ein Infrarotstrahlungsfeld ist und die Empfangs- und Anzeigeeinheit (5) wenigstens zwei Infrarotsensoren aufweist, mit deren Hilfe die räumliche Lage in bezug auf das Strahlungsfeld ermittelt wird.

## Revendications

1. Système de préparation et d'exécution d'une fraisure d'une acétabule dans l'os iliaque naturel avec un angle d'entrée de bassin et un angle d'antéflexion définis sélectionnables, dans le cadre d'une prise en charge endoprothétique d'un patient au niveau des hanches, comprenant :
- un support de patient (1) au moyen duquel un patient peut être couché dans une position spatiale définie,
- un dispositif auxiliaire de calibrage (2) qui est relié directement au support de patient (1) et se compose d'un dispositif de retenue (3) pivotant autour de la verticale et de l'horizontale, le dispositif de retenue (3) pouvant être bloqué dans la position voulue,
- un outil à fraiser (4) pour fraiser l'acétabule, l'outil étant muni dans le champ de vision de l'opérateur,d'une unité électronique de réception et d'affichage (5) qui détecte la position spatiale courante de l'axe longitudinal de l'outil à fraiser (4) par rapport à un signal de localisation sous la forme d'un champ de rayonnement et l'amène à un affichage optique,
- un équipement de repérage (6) qui émet le signal de localisation en direction de l'unité de réception et d'affichage (5), ce qui permet à celle-ci de déterminer la position spatiale de l'outil à fraiser (4) par rapport à l'équipement de repérage (6) et de l'amener à l'affichage,
dans lequel le dispositif de retenue (3) du dispositif auxiliaire de calibrage (2) présente un logement (7) pour l'outil à fraiser (4) dans lequel celui-ci peut être placé après un réglage individuel de l'angle d'antéflexion et de l'angle d'entrée de bassin souhaités grâce au pivotement du dispositif de retenue (3), de sorte que l'outil à fraiser (4) occupe, par son axe longitudinal, l'orientation spatiale souhaitée pour la mise en oeuvre in situ, après quoi cette orientation spatiale est enregistrée entant que position de référence grâce à l'actionnement d'un commutateur d'initialisation (15) sur l'unité de réception et d'affichage (5) par l'enregistrement des valeurs reçues de façon correspondante du signal de localisation provenant de l'équipement de repérage (6), et dans lequel, après réglage de cette valeur de référence, l'outil à fraiser (4), après avoir été retiré du logement (7), peut être ramené à l'orientation spatiale préréglée sur l'acétabule avec un affichage optique.

2. Système selon la revendication 1, dans lequel le support de patient (1) comprend :
- une table d'opération (8) sur laquelle on couche le patient,
- au moins une barre de guidage (9) guidée de façon exactement verticale sur la table d'opération, le dispositif auxiliaire de calibrage (2) étant disposé sur la barre et la barre pouvant être bloquée dans la position souhaitée,
- des pelotes de bassin (10) qui servent à envelopper et maintenir en place le bassin du patient pendant l'opération.

3. Système selon la revendication 1 ou 2, dans lequel le dispositif auxiliaire de calibrage (2) comprend :
- un premier bras de retenue (11) qui peut pivoter autour de l'axe vertical de ladite au moins une barre de guidage (9), et
- un deuxième bras de retenue (12) qui est coudé par l'une de ses extrémités sur le premier bras de retenue (1), et est pivotant indépendamment de celui-ci, et dont l'autre extrémité est réalisée comme un logement (7) pour l'outil à fraiser (4).

4. Système selon l'une quelconque des revendications 1 à 3, dans lequel le dispositif auxiliaire de calibrage (2) présente deux graduations (13, 13') qui affichent les écarts du .logement du dispositif auxiliaire de calibrage (2) par rapport à la verticale et à l'horizontale.

5. Système selon l'une quelconque des revendications 1 à 4, dans lequel l'unité électronique de réception et d'affichage (5) de l'outil à fraiser (4) affiche les écarts courants de son orientation spatiale par rapport à la position de référence enregistrée et affiche,au moyen d'un signal optique,la direction des mouvements de correction à effectuer pour atteindre la position de référence souhaitée.

6. Système selon l'une quelconque des revendications 1 à 5, dans lequel le signal de localisation émis par l'équipement de repérage (6) est un champ de rayonnement infrarouge et l'unité de réception et d'affichage (5) présente au moins deux capteurs infrarouge qui permettent de déterminer la position spatiale par rapport au champ de rayonnement.

## Claims

1. System for preparing and carrying out countersinking of an acetabulum in the natural pelvic bone with selectable, defined pelvic inlet angle and antecurvation angle within the framework of endoprosthetic care of a patient in the hip region, having
- a patient support (1), by means of which a patient can be placed in a defined spatial position,
- a calibration aid (2), which is connected directly to the patient support (1) and consists of a holding device (3) which can be pivoted about the vertical and about the horizontal, wherein the holding device (3) can be locked in the required position,
- a milling tool (4) for countersinking the acetabulum which is fitted with an electronic receiving and display unit (5) in the viewing field of the operator and which records the instantaneous spatial position of the longitudinal axis of the milling tool (4) with reference to a locating signal in the form of a radiation field and displays it optically,
- a direction finder (6), which emits the locating signal in the direction of the receiving and display unit (5), which makes it possible for the latter to determine the spatial position of the milling tool (4) with reference to the direction finder (6) and to display it,
wherein the holding device (3) of the calibration aid (2) has a recess (7) for the milling tool (4), into which the latter can be placed after individual adjustment of the required antecurvation and pelvic inlet angle by pivoting the holding device (3), so that the milling tool (4) with its longitudinal axis occupies the required spatial orientation for use in situ, after which this spatial orientation is stored as a reference position by actuating a set switch (15) on the receiving and display unit (5) by storing the values of the locating signal received accordingly from the direction finder (6), and wherein after adjusting this reference value, the milling tool (4) can be returned to the pre-adjusted spatial orientation on the acetabulum after removal from the recess (7) under optical display.

2. System according to claim 1, in which the patient support (1) comprises:
- an operating table (8), on which the patient is laid,
- at least one guide rod (9) guided exactly vertically on the operating table, and on which the calibration aid (2) is arranged and can be locked in the required position,
- pelvic truss pads (10) which serve to engage around the pelvis of the patient during the operation and hold it in position.

3. System according to claim I or 2, in which the calibration aid (2) comprises:
- a first holding arm (11), which can be pivoted about the vertical axis of the at least one guide rod (9), and
- a second holding arm (12), which is articulated on the first holding arm (1) by its one end and can be pivoted independently of the latter and its other end is designed as a recess (7) for the milling tool (4).

4. System according to one of claims 1 to 3, in which the calibration aid (2) has two scales (13, 13'), which displays the deviations of the recess of the calibration aid (2) from the vertical and the horizontal.

5. System according to one of claims 1 to 4, in which the electronic receiving and display unit (5) of the milling tool (4) displays the instantaneous deviations of its spatial orientation from the stored reference position and displays, by means of an optical signal, the direction of the correction movements to be carried out in order to reach the required reference position.

6. System according to one of claims 1 to 5, in which the locating signal emitted by the direction finder (6) is an infrared radiation field and the receiving and display unit (5) has at least two infrared sensors, with the aid of which the spatial position with reference to the radiation field is determined.
